# EUROPÄISCHE PATENTSCHRIFT

(11) **EP 1 858 459 B1**
(45) Veröffentlichungstag und Bekanntmachung des Hinweises auf die Patenterteilung: **02.03.2011**
(21) Anmeldenummer: 06722984.9
(22) Anmeldetag: 26.01.2006
(51) Int. Cl.: A61F 9/007

(54) **VORRICHTUNG ZUM ABDICHTEN EINER ÖFFNUNG IN EINEM MENSCHLICHEN ODER TIERISCHEN AUGE**
APPARATUS FOR SEALING AN OPENING IN A HUMAN OR ANIMAL EYE
DISPOSITIF POUR ETANCHEIFIER UNE OUVERTURE DANS UN OEIL, CHEZ L'HOMME OU L'ANIMAL

(30) Priorität: 22.02.2005 DE 102005008235
(43) Veröffentlichungstag der Anmeldung: 28.11.2007
(73) Patentinhaber: Thyzel, Reinhardt, 90562 Heroldsberg (DE)
(72) Erfinder: Thyzel, Reinhardt, 90562 Heroldsberg (DE)
(74) Vertreter: Schröer, Gernot H.
(86) Internationale Anmeldenummer: PCT/EP2006/000653
(87) Internationale Veröffentlichungsnummer: WO 2006/089611

(56) Entgegenhaltungen:
- EP-A- 0 216 952
- WO-A-01/68016
- WO-A-97/47247
- US-A- 3 528 410
- US-A- 4 381 007

## Beschreibung

Die Erfindung betrifft eine Vorrichtung zum Abdichten einer Öffnung in einem menschlichen oder tierischen Auge.

Das menschliche Auge oder der Augapfel umfasst einen von einer in Hornhaut (*cornea*), *sclera* und Lederhaut unterteilten Außenhaut umschlossenen Augeninnenraum, der in Lichteinfallsrichtung gesehen in eine vordere Augenkammer, eine hintere Augenkammer und einen Glaskörperraum, in dem der Glaskörper angeordnet ist, aufteilbar ist. Zwischen der vorderen Augenkammer und dem Glaskörper ist die Augenlinse (*lens*) angeordnet, die über die *Zonula-Fasern* an dem an der Lederhaut ausgebildeten *ciliar*-Muskelsystem aufgehängt ist und dadurch in ihrer Konvexität und damit ihrer optischen Brennweite verändert werden kann (Akkomodation). Den Glaskörper umgibt die Netzhaut (*retina*), die über den Sehnerv mit dem Gehirn verbunden ist. Die Hornhaut bildet den vorderen transparenten Bereich der Augenaußenhaut und bildet zusammen mit der in der Augenkammer befindlichen transparenten Flüssigkeit, die überwiegend aus Wasser besteht, der Linse sowie dem Glaskörper ein optisches Abbildungssystem. Das durch dieses optische Abbildungssystem auf der Netzhaut abgebildete Bild wird von der Netzhaut aufgenommen und über den Sehnerv ans Gehirn weitergeleitet.

In dem Augeninnenraum herrscht ein Augendruck oder Innendruck, der größer ist als der Außendruck oder Atmosphärendruck (Normaldruck). Insbesondere steht also die Flüssigkeit in der Augenkammer unter einem erhöhten Innendruck.

In der Ophthalmologie (Augenheilkunde) sind verschiedene chirurgische Eingriffe bekannt, bei denen mit operativen Instrumenten in das Augeninnere eingegriffen wird. Bei diesen intraokularen invasiven Eingriffen wird wenigstens eine Öffnung im Auge in dessen Augenaußenhaut erzeugt, durch die ein Instrument in den Augeninnenraum eingeführt wird.

An dieser Öffnung entsteht nun eine Druckundichtigkeit und daraus folgend eventuell ein Leck für die Augenflüssigkeit. Ein Abfall des Augeninnendrucks sollte aber vermieden werden. Deshalb wird während der Operation über das Operationsinstrument selbst oder über ein separates zweites Instrument Spül- oder Irrigationsflüssigkeit in das Auge eingeführt, um den Druckverlust fortlaufend ausgleichen zu können.

Die dadurch entstehende Flüssigkeitsströmung und die unterschiedlichen Drücke verursachen meistens jedoch Gewebe- oder Zellschädigungen, so dass der Austausch von Flüssigkeit oder, mit anderen Worten, der Volumenstrom oder Durchfluss der Flüssigkeit während der Operation möglichst gering gehalten werden soll.

Eine häufig angewendete intraokulare chirurgische Maßnahme ist das Ersetzen einer natürlichen Augenlinse durch eine künstliche (synthetische) Augenlinse (intraokulare Linse), die in der Regel aus einem im sichtbaren Spektrum transparenten Polymerwerkstoff, insbesondere Acrylglas (PMMA) oder Silikon (Siloxan-Elastomer) besteht. Bei diesem operativen Eingriff wird die natürliche Linse aus ihrem Linsenkapselsack (*capsula lensis*) entfernt (Explantation) und anschließend wird eine intraokulare Linse in den verbliebenen Linsenkapselsack eingebracht (Implantation). Die Explantation der natürlichen Linse erfolgt in der Praxis durch Zerstörung und Austragen des Linsengewebes (Phakolyse), im Allgemeinen durch Phakoemulsifikation, bei der mittels Ultraschall oder mittels mit Laserlicht (Photolyse) erzeugter Schockwellen die Linse emulsifiziert (verflüssigt) und abgesaugt wird. Die Verwendung von Faltlinsen oder injizierbaren Linsen ermöglicht eine Reduzierung des chirurgischen Schnitts auf in der Praxis nur noch 2 mm oder sogar kleiner. Das Ersetzen der natürlichen Augenlinsen durch eine synthetische intraokulare Linse wird derzeit vorrangig zur Behebung einer Katarakt (Linsentrübung oder Star), eingesetzt. Es sind aber auch andere Anwendungsfälle möglich, beispielsweise das Einsetzen einer intraokularen Linse zur Anpassung oder Korrektur der optischen Brennweite, beispielsweise bei Kurzsichtigkeit (Myopie) oder Weitsichtigkeit (Hyperopie), oder nach Unfällen oder Verletzungen der Linse, bei dem der Linsenkapselsack selbst nicht irreparabel beschädigt ist.

Aus US 5,324,281 A ist ein chirurgisches Instrument in Form einer Nadel zum Zerstören von Gewebe bekannt, das zum photolysischen Entfernen von Augenkatarakten bestimmt ist. Dieses bekannte Instrument weist eine Nadel sowie eine Laserfaser und einen Absaugkanal, die jeweils longitudinal und im Innenraum der Nadel bis zu deren freien Ende verlaufen, auf. Am freien Ende der Nadel ist ein Target aus Titan (Ti) angeordnet in einem Abstand zum freien Ende der Laserfaser, wobei die Laserfaser und das Target so zueinander justiert sind, dass das Laserlicht aus dieser Faser auf das Target trifft. Ferner ist an dem freien Ende der Nadel eine schräg und seitlich versetzt angeordnete Gewebeaufnahmeöffnung vorgesehen, in die der Absaugkanal mündet und die unmittelbar benachbart zu dem Target und dem Zwischenraum zwischen dem Laserfaserende und dem Target angeordnet ist. Über eine Absaugpumpe wird in dem Absaugkanal ein Unterdruck (Vakuum) erzeugt, mittels dessen das zu zerstörende Gewebe an die Gewebeaufnahmeöffnung angesaugt wird. Wenn nun das Gewebe an der Gewebeaufnahmeöffnung durch den Unterdruck anliegt, wird das Target mit Laserpulsen aus der Laserfaser beschossen, wobei die Laserpulse eine ausreichende Energie haben, um einen optischen Durchbruch an der Oberfläche des Targetmaterials zu erzeugen. Damit wird eine Stoßwelle erzeugt, die an der Gewebeaufnahmeöffnung auf das dort befindliche Gewebe trifft und dieses in kleine Stücke reißt, die dann durch den Absaugkanal abgesaugt werden. Die Laserpulse haben insbesondere eine Pulsdauer von 8 ns und eine Pulswiederholungsrate von 20 Pulsen pro Sekunde und werden vorzugsweise mit einem Neodym-YAG-Laser erzeugt mit einer Wellenlänge von 1064 nm. Ferner kann in der Nadel zusätzlich ein longitudinal verlaufender Spülkanal zum Leiten von Spülflüssigkeit durch eine seitlich angeordnete Austrittsöffnung vorgesehen sein.

In US 5,906,611 A ist eine Weiterbildung des aus US 5, 324, 282 A bekannten Instruments bekannt, bei der das Target in spezieller Weise stufenförmig ausgebildet ist. Mit einem Neodym-YAG-Laser können Pulse erzeugt werden mit Pulswiederholungsraten zwischen 2 und 50 Pulsen pro Sekunde und . Pulsenergien zwischen 2 und 15 mJ. Die Pulsdauer kann zwischen 8 und 12 ns eingestellt sein. Vorzugsweise ist die Pulswiederholungsrate zwischen 2 und 6 Pulsen pro Sekunde und die Pulsenergie zwischen 6 und 10 mJ eingestellt. Für eine Kataraktoperation werden zwischen 200 und 800 Pulse oder Schüsse verwendet.

Ein ähnlich wie in US 5,324,282 A und US 5,906,611 A aufgebautes Laserhandstück wird zusammen mit einem digitalen Steuer- und Versorgungsgerät, das einen Laser für die Laserpulse sowie eine Venturi-Pumpe zum Absaugen der Gewebeteile umfasst, bereits seit Jahren erfolgreich unter der Bezeichnung "Lyla/Pharo" von der A.R.C. Laser GmbH angeboten und wurde in einer Vielzahl von Operationen erfolgreich angewendet. Dabei wird über das Laserhandstück abgesaugt und die Irrigation mit einer elektrolytischen Irrigations- oder Spüllösung (BSS) erfolgt über ein zweites Instrument in einer bimanuellen Technik. Für die eigentliche Augenoperation unter Verwendung dieser bekannten Vorrichtung werden unterschiedliche Operationstechniken angewendet.

Bei einer Operation mit den vorbeschriebenen Laserhandstücken wird die Nadel in die Inzision am Auge eingebracht, wobei das Gewebe die Nadel fest umschließt und dadurch die Nadel gegen die die Öffnung umschließende Innenrandfläche der Außenhaut abdichtet.

Bei einer Phakolyse mit Ultraschall kommen Ultraschall-Instrumente zum Einsatz mit einer piezoelektrisch in axiale Schwingungen versetzten Ultraschallnadel, die durch die Inzision in das Auge eingeführt wird. Diese Ultraschallnadel vibriert mit einer Ultraschallfrequenz, die beispielsweise im Bereich von Kilohertz, beispielsweise 40 kHz, liegt. Ein solches Ultraschallphakoemulsifikationssystem der A.R.C. Laser GmbH ist unter der Bezeichung "Pharo" bekannt.

Aufgrund der hohen mechanischen Energie ist ein Abdichten der Ultraschallnadel unmittelbar an dem umgebenden Gewebe der Augenaußenhaut an der Inzision nicht möglich, da das Hautgewebe bei Berührung mit der Ultraschallnadel zerstört oder verletzt würde (thermische Gewebeschäden, Verbrennung). Die Inzision wird deshalb bei Ultraschall-Phakolysen zur Vermeidung eines Kontakts oder der Reibung der Ultraschallnadel an dem Hautgewebe in der Regel größer ausgebildet als bei Lasernadeln (typischerweise 2,6 mm bis 3,2 mm bei Ultraschall und bei Laser typischerweise 1,4 mm) .

Zugleich wird eine Hülse (sleeve) aus einem weichelastischen und schwingungsdämpfenden Werkstoff, beispielsweise einem Silikon (Siloxankautschuk), um die Ultraschallnadel angeordnet, die die Nadel vollständig umgibt und den Außenhautgeweberand um die Inzision vor der schwingenden Nadel schützt. Zwischen der Nadel und der Hülse wird durch die Inzision Irrigationsflüssigkeit ins Augeninnere geführt. Die Hülse wird dabei unter Druck gesetzt und nach außen aufgebläht oder gedrückt und dichtet dadurch zusätzlich die Öffnung nach außen gegen das an der Hülse seitlich anliegende Hautgewebe ab. Bei dieser Abdichtung liegt die Hülse mit ihrem Umfang an den die Öffnung umgebenden Seitenflächen der Haut an, so dass die Länge der Abdichtung der Dicke der Außenhaut an dieser Stelle entspricht. Eine Leckage von Augenflüssigkeit zwischen Hülse und Geweberand kann in der Praxis dennoch nicht vollständig verhindert werden, unter anderem auch da durch die Bewegungen des Operateurs immer wieder Zwischenräume zwischen Augenaußenhaut und Hülse entstehen.

Bei der Augenlinsenextraktion soll die Irrigations-Flüssigkeit idealerweise nur das Volumen des abgesaugten Linsengewebes ersetzen bzw. den beim Absaugen entstehenden Unterdruck wieder ausgleichen. Dies wird in der Praxis aufgrund der beschriebenen Undichtigkeiten an den Öffnungen nicht erreicht. Typischerweise beträgt der Unterdruck beim Absaugen von Gewebe wie bei der Linsenextraktion 700 bis 800 mbar, also 200 bis 300 mbar unter Normaldruck oder Atmosphärendruck. Dieser Differenzdruck ist für das Auge vergleichsweise hoch und macht eine hohe Dichtigkeit oder Abdichtung der Öffnung am Auge erforderlich.

Um eine ausreichende Augenkammerstabilität und einen ausreichenden Augeninnendruck aufrecht zu erhalten, wird deshalb während einer Augenoperation das Volumen der Flüssigkeit im Auge nachgeregelt, indem eine Druckregelung im Flüssigkeitszuführsystem und in den Schläuchen sehr schnell den Druck im Auge durch Zuführen von Flüssigkeit oder Stellen des Volumenstroms auf den Sollwert nachregelt. Dies macht einen vergleichsweise hohen regeltechnischen Aufwand erforderlich.

Ein weiterer bekanntet intraokularer chirurgischer Eingriff ist die Vitrektomie, bei der der Glaskörper teilweise mittels eines durch eine Öffnung im Auge eingeführten Schneidinstruments entfernt wird. Bei dieser Operation ist eine besonders hohe Dichtigkeit der Öffnung im Auge erforderlich bzw. ein niedriger Infusionsdruck (typischerweise 15 bis 20 mbar unter Atmosphärendruck), um die Netzhaut nicht zu beschädigen. Die Inzisionsgröße ist bei dieser Operation typischerweise 0,6 mm.

Aus EP 0 216 952 ist eine Einrichtung zur Drainage subretinaler Flüssigkeirsansammlungen für die Anwendung in der Augenheilkunde bekannt mit einem Saugnapf, welcher von außen an den Augapfel angesaugt wird, und einer elektrisch isolierten Nadelelektrode, welche durch ein automatisches Antriebsaggregat angetrieben wird. Der Saugnapf verhindert eine Eindellung der Sclera während des Einstechens der Nadelspitze in die Sclera.

Die US 3,528,410 offenbart eine Vorrichtung zum Behandeln einer Retinaablösung mittels Ultraschall, wobei eine Ultraschallnadel mit einer umgebenden Hülse durch die Hornhaut gestochen wird und mit einem im Längsschnitt widerhakenähnlichen Element innen die Öffnung in der Hornhaut abdichtet.

Der Erfindung liegt nun die Aufgabe zugrunde, eine neue Vorrichtung zum Abdichten einer Öffnung in einem menschlichen oder tierischen Auge anzugeben, bei der die genannten Nachteile beim Stand der Technik zumindest teilweise gelindert oder ganz vermieden werden.

Diese Aufgabe wird gemäß der Erfindung gelöst mit den Merkmalen des Patentanspruches 1.

Die Erfindung beruht auf der Überlegung, die Öffnung im Auge bei ophthalmologischen invasiven Eingriffen nicht innerhalb der Öffnung abzudichten, sondern auf der Außenfläche (oder: nach außen gerichteten Oberfläche) des Augengewebes um die Öffnung herum. Dadurch wird, wie auch Versuche zeigten, eine deutlich bessere Abdichtung mit deutlich niedrigeren Druck- und Flüssigkeitsverlusten erreicht. Diese Abdichtung ist deutlich weniger abhängig von der Gestalt und Größe der Öffnung als beim Stand der Technik und ermöglicht eine hervorragendes Abdichten auch länglicher Inzisionen sowie großer wie kleiner Inzisionen (Mikroinzisionen). Während die zur Verfügung stehende Abdichtfläche zum Abdichten einer Augenöffnung beim Stand der Technik durch die Dicke des Augengewebes an dieser Stelle begrenzt ist und außerdem die Abdichtung sehr empfindlich gegenüber Bewegungen des Operationsinstruments relativ zur Öffnung ist, ist die Abdichtfläche gemäß der Erfindung in weiten Grenzen in Größe und Form einstellbar und auch im Abstand von der Öffnung aufsetzbar, so dass Veränderungen der Form oder Größe der Öffnung die Abdichtung nicht beeinflussen.

Bei einer Operation zur Extraktion von Augengewebe, insbesondere Augenlinsengewebe, kann dadurch insbesondere der Absaugdruck oder der Absaugvolumenstrom bei der Aspiration deutlich erhöht werden und somit der Anteils der Aspiration bei dem Gewebeaustrag oder der Extraktion deutlich größer, beispielsweise auf 70 %, und der Anteil der Gewebezerstörung durch den Energieeintrag durch Laserpulse oder Ultraschallschwingungen erheblich niedriger eingestellt werden. Der höhere Sog ermöglicht nämlich das Absaugen größerer Gewebeteile, so dass das Gewebe vor dem Absaugen nicht mehr so stark verkleinert werden muss. Außerdem wird aufgrund der größeren Dichtigkeit und des praktisch geschlossenen druckdichten Systems oder Irrigations/Aspirationskreislaufs die Durchflussmenge und das benötigte Flüssigkeitsvolumen kleiner und die Regelung der Irrigation einfacher. Insbesondere auch bei Ultraschall-Instrumenten kann die Operation atraumatischer mit geringerer Ödem- oder Quetschungsneigung und sicherer mit geringerem Infektionsrisiko erfolgen und eine Verbrennung des Augengewebes weitgehend vermieden werden.

Vorteilhafte Ausgestaltungen und Weiterbildungen der Vorrichtung gemäß der Erfindung ergeben sich aus den vom Anspruch 1 abhängigen Ansprüchen.

Die Erfindung wird im Folgenden anhand von Ausführungsbeispielen weiter erläutert. Dabei wird auch auf die Zeichnungen Bezug genommen, in deren in der Liste der Figuren
- FIG 1: ein Abdichtelement mit einem aufgenommenen Operationsin- strument bei einem operativen Eingriff an einem Auge in per- spektivischer Darstellung,
- FIG 2: die Anordnung gemäß FIG 1 in einer geschnittenen Darstel- lung,
- FIG 3: die Anordnung gemäß FIG 2 in einem vergrößerten Ausschnitt,
- FIG 4: das Abdichtelement gemäß FIG 1 bis 3 allein in einer Schnitt- darstellung,
- FIG 5: das Abdichtelement gemäß FIG 4 in einer perspektivischen Darstellung,
- FIG 6: eine weitere Ausführungsform eines Abdichtelements mit ge- genüber FIG 4 abgewandeltem Anlagebereich in einer Schnitt- darstellung,
- FIG 7: das Abdichtelement gemäß FIG 6 in einer perspektivischen Darstellung,
- FIG 8: eine weitere Ausführungsform eines Abdichtelements in einer Schnittdarstellung,
- FIG 9: eine weitere Ausführungsform eines Abdichtelements mit ei- nem Laseroperationsinstrument beim Einsatz am Auge in einer perspektivischen Darstellung,
- FIG 10: die Anordnung gemäß FIG 9 in einer Schnittdarstellung,
- FIG 11: das in FIG 9 und 10 eingesetzte Abdichtelement allein in einer Schnittdarstellung,
- FIG 12: das Abdichtelement gemäß FIG 11 in einer perspektivischen Darstellung,
- FIG 13: ein gemäß dem Stand der Technik ausgebildetes Abdichtele- ment im Einsatz am Auge und
- FIG 14: die Anordnung gemäß dem Stand der Technik gemäß FIG 13 in einer Schnittdarstellung.
- FIG 15: ein Abdichtelement gemäß dem Stand der Technik gemäß FIG 13 und 14 in einer vergrößerten Schnittdarstellung,
- FIG 16: das Abdichtelement gemäß dem Stand der Technik gemäß FIG 15 in einer perspektivischen Darstellung,
- FIG 17: ein weiteres Abdichtelement gemäß der Erfindung in einer Schnittdarstellung,
- FIG 18: ein weiteres Abdichtelement in einer Schnittdarstellung und
- FIG 19: das Abdichtelement gemäß FIG 18 in einer perspektivischen Darstellung.

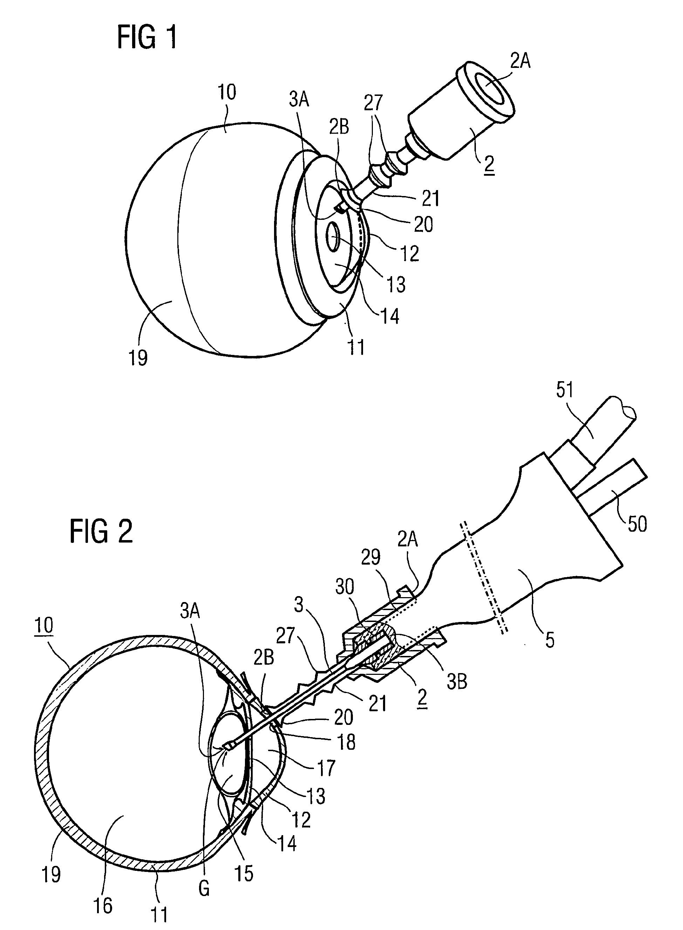

Einander entsprechende Teile und Größen sind in den FIG 1 bis 19 mit denselben Bezugszeichen versehen.

FIG 1 bis 3 zeigen schematisch den Einsatz eines Operationsinstrumentes 3 zur Linsenextraktion bei einer Katarakt-Augenoperation.

Das Auge ist mit 10 bezeichnet. In dem von der Sclera 11, Hornhaut (Cornea) 12 und Lederhaut 19 umschlossenen Augeninnenraum sind der Glaskörper 16 und vor dem Glaskörper 16 die Augenlinse 15 angeordnet. Die Augenlinse 15 ist von dem nicht bezeichneten Kapselsack umschlossen und über die Zonularfasern an dem Ciliarmuskel an der Sclera 11 aufgehängt. Vor der Augenlinse 15 ist die Iris (oder: Regenbogenhaut) 14, die die Pupille 13 umschließt und deren Größe einstellt, angeordnet. Vor der Iris 14 und der Pupille 13 befindet sich die vordere Augenkammer 17, die mit Augenflüssigkeit gefüllt ist und von der transparenten Hornhaut 12 nach vorne begrenzt wird.

Eine Katarakt-Operation umfasst nun in der Regel die folgenden Verfahrensschritte:

Zunächst wird mittels eines chirurgischen Instruments, beispielsweise einer Kanüle, der vorderer Kapselsack der Augenlinse 15 eröffnet, wobei eine in der Regel 4,5 mm bis 5,5 mm große Öffnung erzeugt wird *(Capsulorrhexis).* Durch Einbringen einer Irrigationsflüssigkeit, z.B. BSS, wird die Augenlinse 15 vom Kapselsack gelöst und dadurch mobilisiert (Hydrodisektion).

Sodann werden bei einer Operationstechnik mit nur einem Instrument (monomanuelle Technik) nur eine Öffnung (Inzision, Schnitt) 18 in der Hornhaut *(cornea),* insbesondere am *Limbus,* vorzugsweise am Übergang von Hornhaut 12 zu Sclera 11, und bei einer anderen Operationstechnik mit zwei Instrumenten (bimanuelle Technik) werden, in der Regel an entgegengesetzten Seiten der Hornhaut *(cornea),* insbesondere am *Limbus,* zwei Inzisionen erzeugt. Im Fall von nur einer Inzision wird ein Operationsinstrument eingesetzt, das eine integrierte Irrigation (oder: Zuführung von Irrigationsflüssigkeit) und Aspiration (oder: Absaugen von Gewebe und Flüssigkeit) aufweist. Im Fall von zwei Inzisionen wird durch eine Inzision ein Operationsinstrument mit integrierter Aspiration eingeführt und durch die weitere Inzision ein separates Irrigationsinstrument zur Irrigation.

Es wird nun Irrigationsflüssigkeit, im Allgemeinen ebenfalls BSS, in den Kapselsack eingespült und durch den dadurch aufgebauten Druck insbesondere verhindert, dass die hintere Wandung des Kapselsackes zu nahe an das Operationsinstrument 3 gelangt, und zugleich kann zusätzlich der Kapselsack gereinigt werden.

In FIG 1 bis 3 ist die Operationsphase gezeigt, bei der bereits eine Öffnung 18 in der Augenaußenhaut erzeugt ist für eine monomanuelle Technik. Bei einer bimanuelle Technik kann man sich einfach eine weitere Öffnung an der entgegengesetzten Seite denken, durch die dann das Irrigationsinstrument hindurchgeführt wird.

Durch die Öffnung 18 ist ein an sich bekanntes mit Ultraschallschwingungen arbeitendes Operationsinstrument 3 hindurch geführt, das in den Linsenkapselsack mit seinem freien Ende 3A eindringt und die im Kapselsack befindliche Augenlinse 15 aus dem Kapselsack extrahiert. Das Operationsinstrument 3 ist nadel- oder kanülenförmig ausgebildet und weist eine nicht näher dargestellte Aspirationsöffnung an seinem freien Ende 3A sowie einen von der Aspirationsöffnung durch das Innere des Operationsinstruments 3 verlaufenden Aspirations- oder Ansaugkanal auf.

Durch ein an einem verbreiterten Anschlussbereich am anderen Ende 3B des Operationsinstruments 3 lösbar angeschlossenes oder angekoppeltes Handstück 5, insbesondere mittels eines im Handstück 5 befindlichen piezoelektrischen Schwingungsantrieb (Piezoantrieb), wird das Operationsinstrument 3 in Schwingungen im Ultraschallspektrum, typischerweise oberhalb 20 kHz, beispielsweise um die 40 kHz, versetzt und zerstört durch die dadurch eingebrachte Energie das Gewebe der Augenlinse 15.

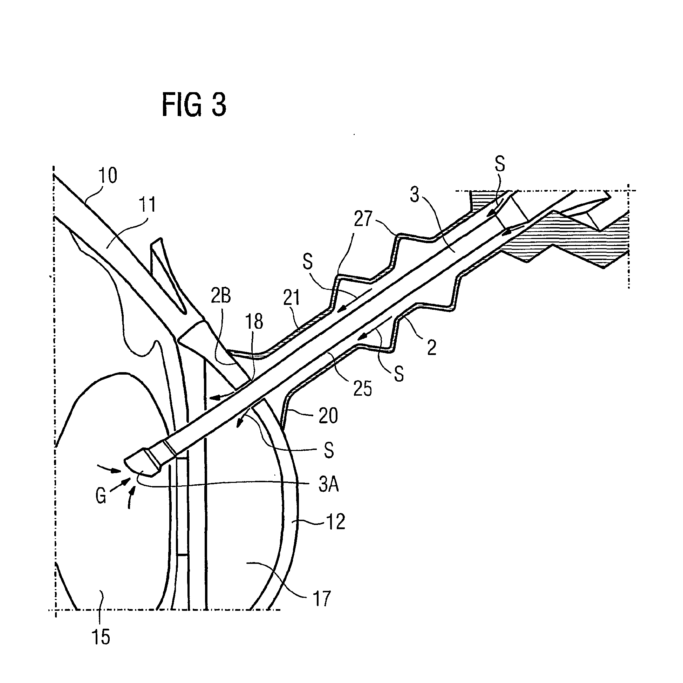

Das Handstück 5 weist seinerseits einen internen Aspirationskanal auf, der den Aspirationskanal im Operationsinstrument 3 mit einem Aspirationsanschluss (oder: Aspirationsschlauch) 50 am Handstück 5 verbindet. Der Aspirationsanschluss 50 wiederum ist mit einer Saug- oder Fördereinrichtung, insbesondere einer Pumpe, zum Erzeugen eines Unterdrucks typischerweise im Grobvakuumbereich und/oder im Bereich eines Absolutdrucks von 700 mbar bis 800 mbar, verbunden. Dadurch wird das zerstörte oder zersetzte Gewebe der Augenlinse 15 in der Regel zusammen mit im Kapselsack befindlicher Flüssigkeit in der in FIG 3 mit G bezeichneten Absaugrichtung infolge des Unterdrucks durch die Öffnung am Ende 3A des Operationsinstruments 3 und die Aspirationskanäle im Operationsinstrument 3 sowie im Handstück 5 und den Aspirationsanschluss 50 abgesaugt.

Außerdem weist das Handstück 5 einen weiteren Anschluss 51 auf, der an ein Kabel angeschlossen ist zum Zuführen elektrischer Energie für den Piezoantrieb.

Das dargestellte Handstück 5 weist also eine integrierte Aspiration auf.

Die Irrigation wird in einer Ausführungsform mittels eines gesonderten Handstücks bewerkstelligt. In einer anderen Ausführungsform weist das Handstück 5 zusätzlich einen weiteren, nicht dargestellten Irrigationsanschluss und einen internen Irrigationskanal zum Zuführen von Irrigationsflüssigkeit (oder: Spül- und/oder Kühlflüssigkeit), beispielsweise BSS, auf. Aus einem Irrigationsausgang, in den der Irrigationskanal mündet, strömt die Irrigationsflüssigkeit dann aus dem Handstück 5 aus und durch den Zwischenraum zwischen der Außenwand des durch den Durchgang 25 geführten Operationsinstrument 3 und der den Durchgang 25 umschließenden Wandung des Abdichtelements 2 und anschließend durch die Öffnung 18 im das Operationsinstrument 3 umgebenden äußeren Bereich in die vordere Augenkammer 17 und den Kapselsack nach.

Dieses Nachführen von Irrigationsflüssigkeit dient dazu, den durch die Aspiration verursachten Druck- und Materialverlust im Augeninneren auszugleichen.

Das Operationsinstrument 3 ist nun in einem Abdichtelement 2 angeordnet und von diesem umhüllt. Das Abdichtelement 2 umfasst einen Aufnahmebereich 22 mit einem, beispielsweise zylindrischen, Aufnahmeraum 26, der sich am Ende 2A öffnet und dort von einem Anschlussflansch 24 umgeben ist, der weiter nach außen ragt als die übrige Wandung des Aufnahmebereichs 22. Der Aufnahmebereich 22 ist über einen Übergangsbereich 23 mit einem Rüsselbereich 21 verbunden, wobei der Durchmesser vom Aufnahmebereich 22 zum Rüsselbereich 21 über den Übergangsbereich 23, beispielsweise gestuft, abnimmt. Zum Übergangsbereich 23 hin ist eine konische Verjüngung zur besseren Flussdynamik gebildet. Innerhalb des Rüsselbereichs 21 und des Übergangsbereichs 23 ist ein, beispielsweise zylindrischer, Durchgang 25 von im Wesentlichen konstantem Durchmesser, der kleiner ist als der Durchmesser des Aufnahmebereichs 26, ausgebildet. Dieser Durchgang 25 mündet in einem Abdichtbereich 20 am vorderen freien Ende 2B nach außen.

In dem Aufnahmebereich 26 des Abdichtelements 2 ist das Ende des Handstücks 5 und der Anschlussbereich 30 des Operationsinstruments 3 aufgenommen. Ein Gewinde 29 im Aufnahmebereich 26 wird dazu auf ein Außengewinde des Handstücks 5 aufgedreht, so dass eine Schraubverbindung realisiert ist.

Durch den Durchgang 25 des Abdichtelements 2 verläuft das (übrige, nadelförmige) Operationsinstrument 3 und ragt mit seinem freien Ende 3A und einem anschließenden Bereich aus dem Abdichtelement 2 heraus und ist somit mit seinem Ende 3A für die Operation verfügbar.

Das Abdichtelement 2 besteht zumindest im Bereich des Rüssels 21 aus einem elastischen Material, beispielsweise aus einem oder auf Basis von einem natürlichen oder synthetischen Kautschuk oder Elastomer und/oder einem thermoplastischen Elastomer. Ein besonders geeignetes Material ist Silicon oder Silicongummi (Siloxankautschuk) oder ein Material mit Silicon, insbesondere ein schwingungsdämpfendes Material wie ein Gemisch mit Elastomer, insbesondere Siloxan-Elastomer, Collagen und Wasser, welches aus WO2004/022999A1 bekannt ist.

Um dem Operateur die Möglichkeit zu geben zu erkennen, wo das im Allgemeinen farblose und transparente Silicongummi des Abdichtelements 2 auf der transparenten Hornhaut 12 anliegt, kann in einer besonderen Ausführungsform das Abdichtelement 2 im Bereich des Abdichtbereichs 20 eingefärbt sein.

Die Wandstärke im Aufnahmebereich 22 und im Übergangsbereich 23 ist vorzugsweise deutlich größer als im Rüsselbereich 21, so dass der Rüsselbereich 21 deutlich verformbarer oder flexibler ist als der Aufnahmebereich 22. Typische Wandstärken für Aufnahmebereich 22 und Übergangsbereich 23 sind größer als 0,7 mm, während die Wanddicke des Rüsselbereichs 21 typischerweise zwischen 0,2 mm und 0,4 mm gewählt wird.

Durch die in FIG 1 bis 3 dargestellte faltenbalgartige Ausbildung des Rüssels 21 mit einer, zwei oder mehreren umlaufenden nach außen ragenden Falten 27 kann der Rüssel 21 noch leichter axial gestaucht werden

Am Ende des Rüssels 21 des Abdichtelements 2 an dessen Ende 2B ist ein Abdichtbereich 20 ausgebildet, der gemäß FIG 1 bis 5 trompetenförmig aufgeweitet ist und an seiner Stirnseite eine ringförmige Anlagefläche als Abdichtfläche bildet.

Wenn nun der Operateur das Handstück 5 mit dem Operationsinstrument 3 durch die Öffnung 18 nach innen zum oder im Kapselsack oder zur Augenlinse 15 zu bewegt, so drückt er zugleich den Rüssel 21 mit seinem Abdichtbereich 20 gegen die äußere Oberfläche der Hornhaut 12 und/oder Sclera 11. Die anliegende ringförmige Abdichtfläche am Abdichtbereich 20 ist von der Öffnung 18 beabstandet und umgibt die Öffnung 18 vollständig, Dadurch dichtete der Abdichtbereich 20 die Öffnung 18 ringsum gegen die äußere Oberfläche der Hornhaut 12 und/oder Sclera 11 ab.

Durch die elastischen Verformbarkeit oder reversible Stauchbarkeit des Rüssels 21 bei axialer Druckbelastung kann der Rüssel 21 vom Operateur unter einem ausreichenden Anpressdruck gegen die Augenaußenhaut gedrückt werden, um die erforderliche druckdichte Abdichtung der Öffnung 18 zu erreichen. Aufgrund der vergleichsweise großen Verformwege des Rüssels 21 und der dadurch bewirkten relativ großen elastischen Rückstellkräfte im Rüssel 21 einerseits und deren räumlicher Entfernung von der Öffnung 18 andererseits ist die Abdichtung relativ unempfindlich gegenüber einer Positionsänderung des Operationsinstrument 3 mit dem Rüssel 21 sowohl in lateraler als auch axialer Richtung. Die Abdichtfläche des Abdichtbereichs 20 bleibt auch bei Änderungen der Form der Öffnung 18 durch die Bewegungen des Operationsinstrument 3 dennoch vollständig auf der Augenaußenhaut (hier 11 oder 12) aufliegen.

Die Wandung des Rüssels 21 im Abdichtbereich 20 wirkt ferner in einer Ausführungsform mit integrierter Irrigation durch die elastischen Rückstellkräfte nach innen in ausreichendem Maße dem durch die Irrigationsflüssigkeit aufgebauten Innendruck im Rüssel 21 entgegen. Ein Flüssigkeitsleck für die durch die Öffnung 18 strömende Irrigationsflüssigkeit um die Öffnung 18 wird somit zuverlässig vermieden.

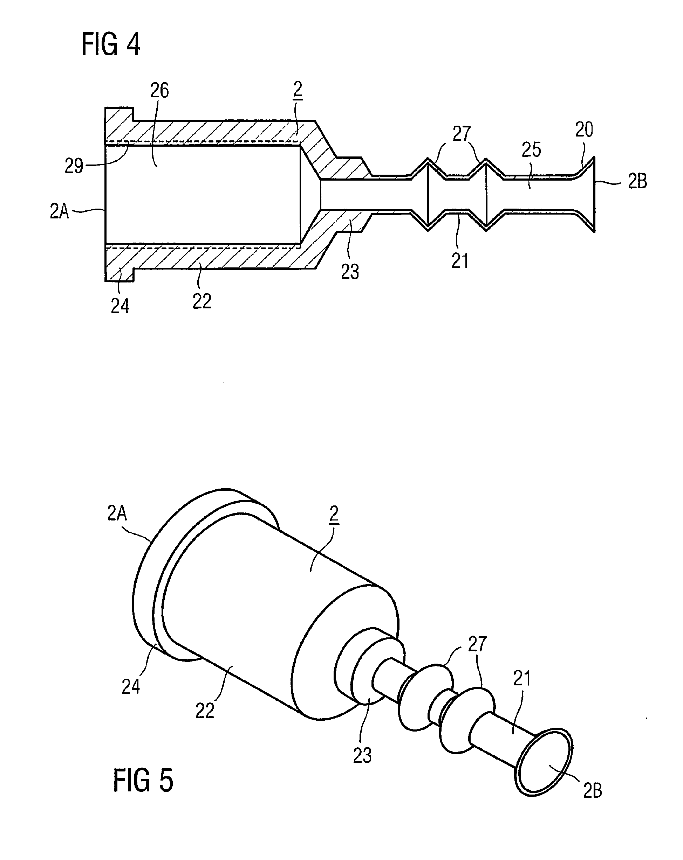

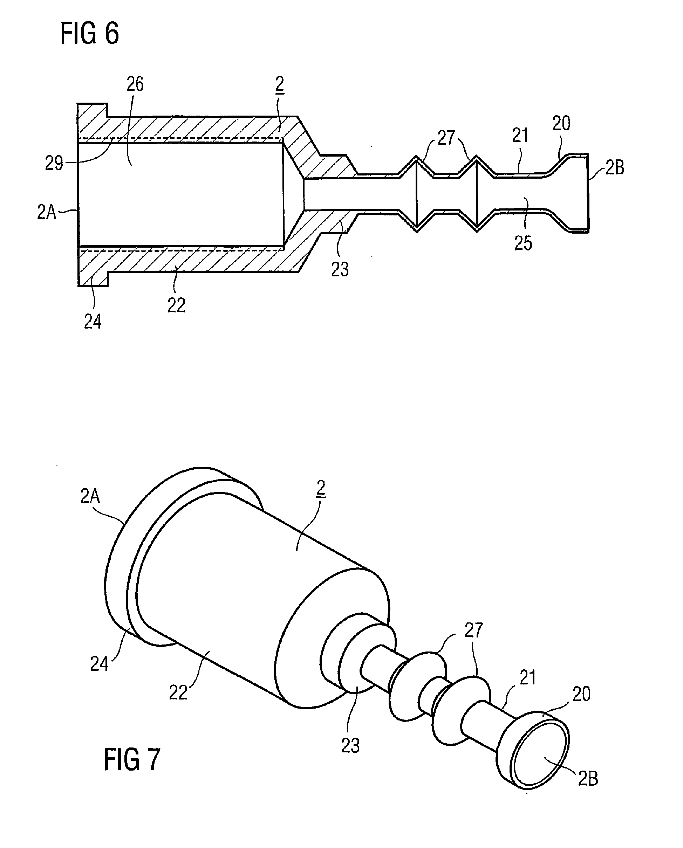

Die FIG 6 und 7 zeigen ein Abdichtelement, das ähnlich aufgebaut ist wie das Abdichtelement gemäß FIG 4 und 5 und nur im Abdichtbereich 20 eine glocken- oder domförmige Gestalt aufweist und nicht eine trompetenförmige Aufweitung. Während also der trompetenförmige Abdichtbereich 20 in den FIG 4 und 5 nach außen oder konkav gekrümmt ist und in seinem Durchmesser bis zum Ende 2B kontinuierlich zunimmt, ist in FIG 6 und 7 der Abdichtbereich 20 nach innen oder konvex gekrümmt und nimmt in seinem Durchmesser zu mit einer zum Ende 2B hin abnehmenden Steigung oder Zunahmerate, wobei der Durchmesser dann am Ende 2B über einen Teilbereich konstant bleibt. In FIG 6 und 7 ist also der Abdichtbereich 20 ähnlich wie eine Saugglocke ausgebildet. Es sind natürlich auch viele andere Formen des Abdichtbereichs 20 möglich, beispielsweise Blasenform oder die Form von Dichtlippen etc.

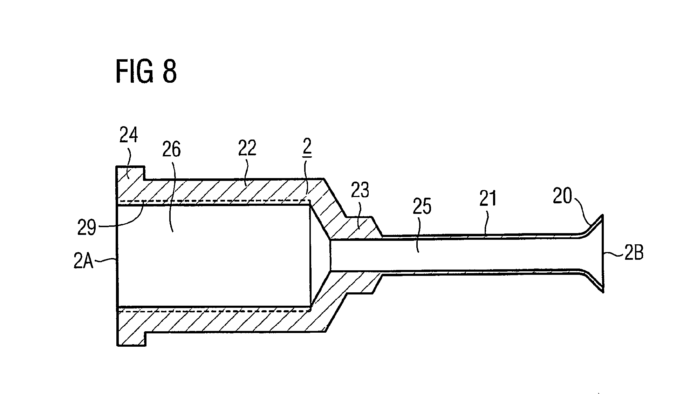

FIG 8 zeigt eine weitere Ausführungsform eines Abdichtelements 2, bei dem im Unterschied zu FIG 4 und 5 die beiden Falten 27 entfallen.

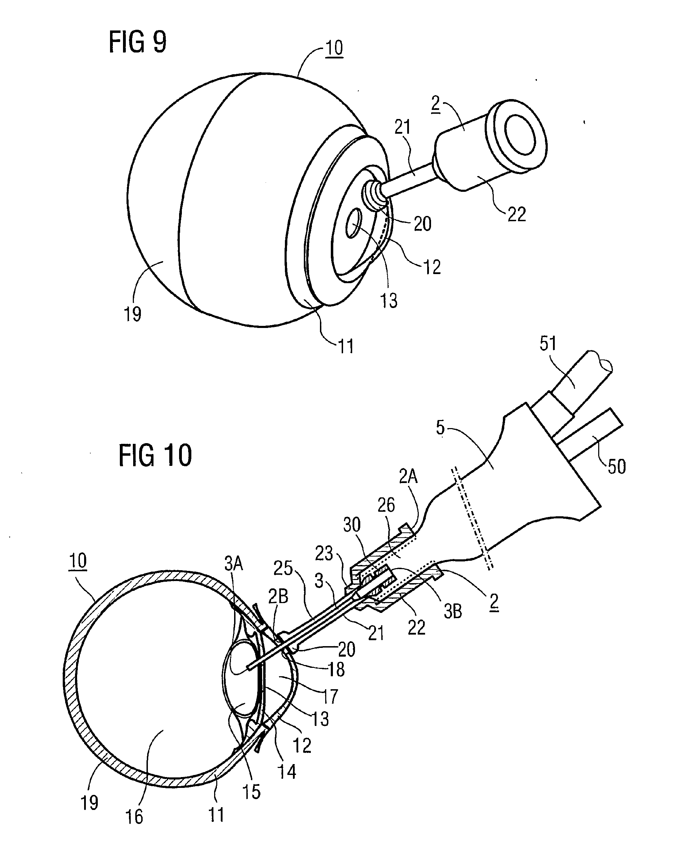

FIG 9 und 10 zeigen ein Abdichtelement 2, das aus dem Abdichtelement gemäß FIG 6 und 7 abgeleitet ist durch Weglassen der Falten 27. Das Abdichtelement 2 ist am Auge angesetzt und sein Abdichtbereich 20 umgibt die Öffnung 18 zum Abdichten des Augeninnenraums gegen den Außenraum.

Das Operationsinstrument 3 in FIG 9 und 10 ist ein an sich bekanntes Laser-Operationsinstrument, beispielsweise ein eingangs genanntes Laserhandstück der A.R.C. Laser GmbH oder ein gemäß der eingangs genannten US 5, 324, 282 A oder US 5,906,611 A aufgebautes Instrument, bei dem die Augenlinse sukzessive durch auf ein Target auftreffende Laserpulse (z.B. Pulsdauer 2 bis 10 ns, bis zu 20 Pulse pro s) und dadurch erzeugte Schockwellen photolysisch abgebaut und dann abgesaugt wird.

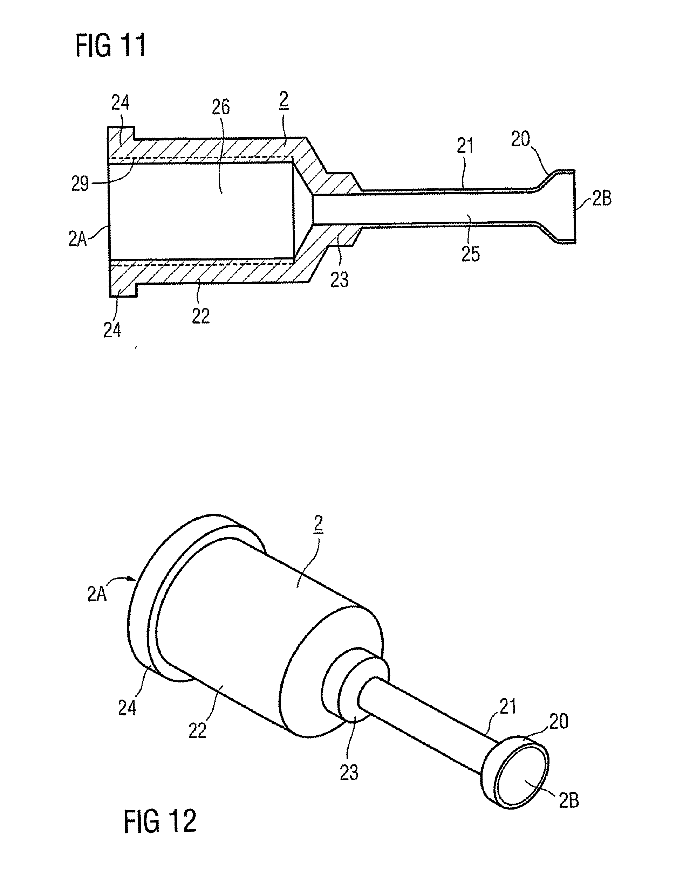

FIG 11 und 12 zeigen das in FIG 9 und 10 verwendete Abdichtelement 2 nochmals in einer detaillierteren Darstellung.

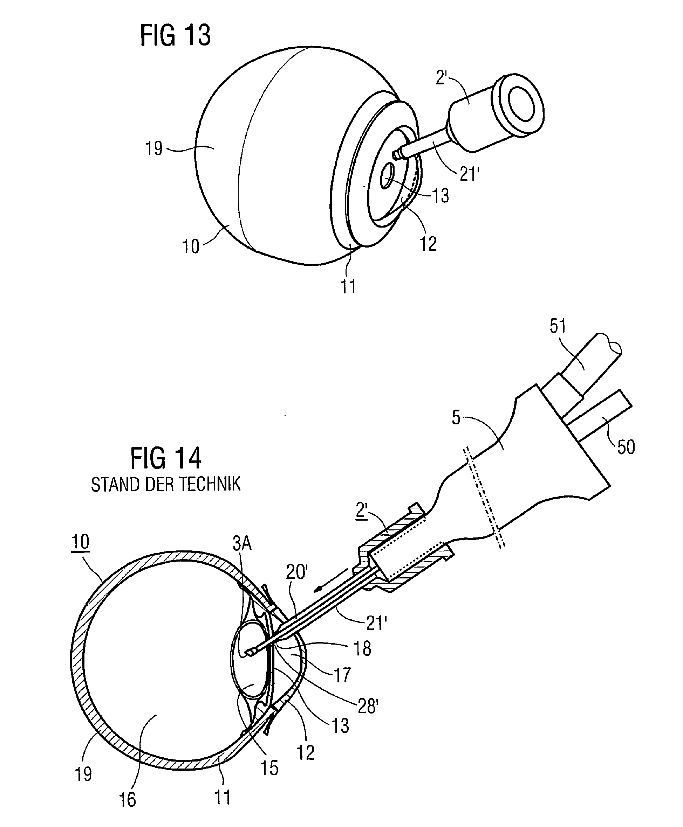

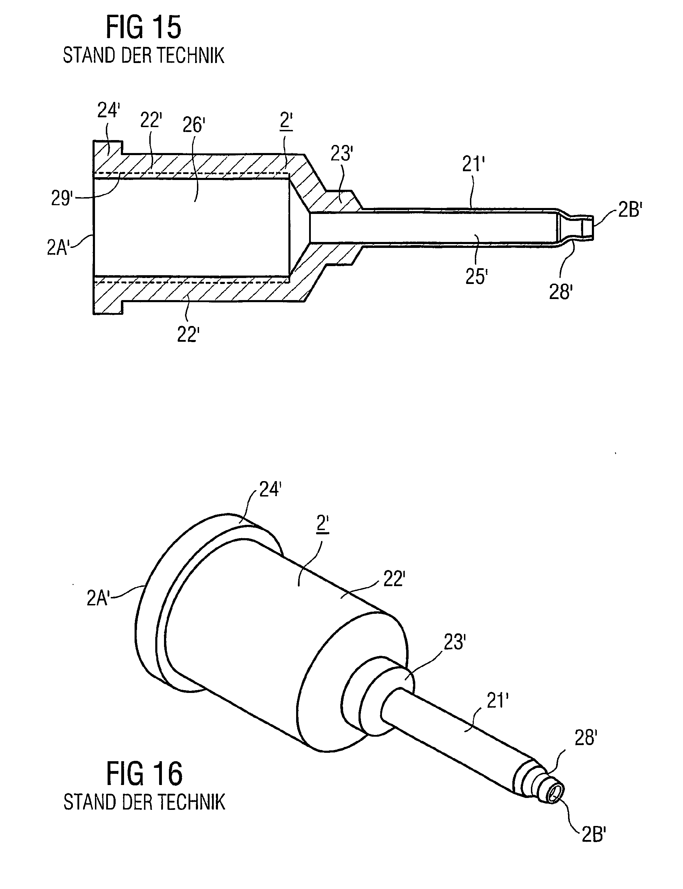

Die FIG 13 und 14 zeigen eine monomanuelle Technik mit einem Ultraschall-Operationsinstrument 3, das von einem Abdichtelement 2' gemäß dem Stand der Technik, das auch Sleeve bezeichnet wird, umgeben ist zum Schutz des umliegenden Augengewebes vor dem vibrierenden Operationsinstrument 3. Das bekannte Abdichtelement 2' ist in FIG 15 und 16 nochmals vergrößert dargestellt und unterscheidet sich von den in FIG 1 bis 10 dargestellten Abdichtelementen 2 gemäß der Erfindung in der Ausgestaltung des Abdichtbereichs gegenüber dem Abdichtbereich 20 bei der Erfindung. Bei den übrigen entsprechenden Bereichen sind die Bezugszeichen nur mit einem Strich versehen, um die Entsprechung zu verdeutlichen. Der Durchgang 25' im Rüsselbereich 21' des Abdichtelements 2' mündet in dem Endbereich 28' am vorderen freien Ende 2B' nach außen. Im Endbereich 28' verjüngt sich nun beim Stand der Technik der Durchmesser des Rüsselbereichs 21' zum Ende 2B' hin nach Art eines Glasflaschenkopfes, wobei dort Austrittöffnungen für die Irrigationsflüssigkeit S sein können. Das bekannte Abdichtelement 2' wird mit der zylindrischen Außenfläche des Rüsselbereichs 21' durch die Öffnung 18 eingeführt, wobei der sich verjüngende Endbereich 28' als Einführhilfe dient.

Im Unterschied zum Stand der Technik gemäß FIG 13 bis 16 wird gemäß der Erfindung die Öffnung 18 in der Hornhaut 12 und/oder Sklera 11 nicht (ausschließlich) an der Innenfläche oder dem umlaufenden Rand der Öffnung 18 abgedichtet, sondern an der Außenfläche der Sklera 11 und/oder Hornhaut 12 mittels einer die Öffnung 18 vollständig umgebenden Abdichtfläche.

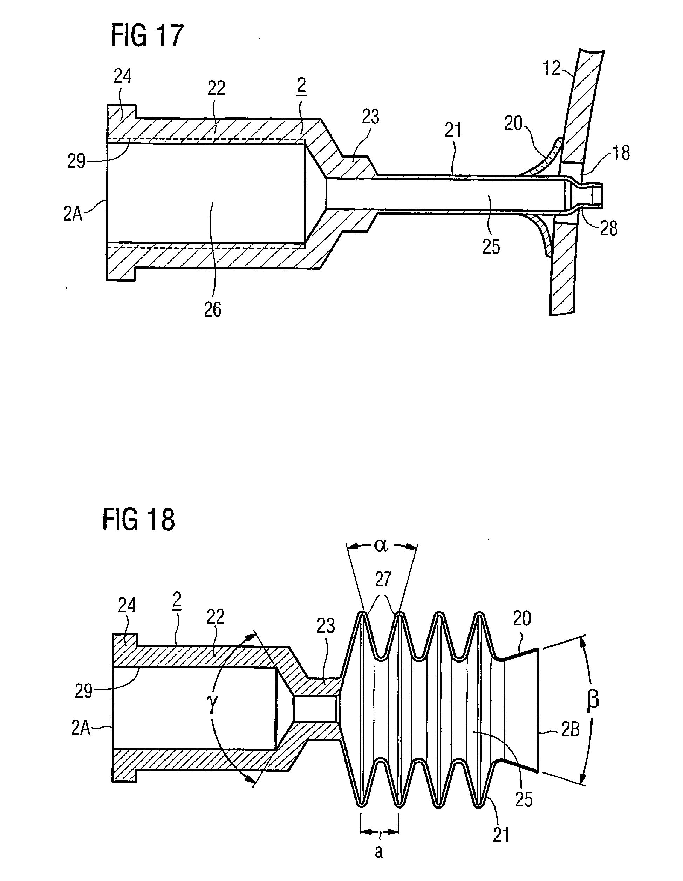

FIG 17 zeigt eine weitere Ausführungsform eines Abdichtelements 2 gemäß der Erfindung, bei der der Abdichtbereich 20 nicht am Ende 2B des Abdichtelements 2 angeordnet ist, sondern an der Außenwandung des Rüsselbereichs 21 ausgebildet oder angeformt ist nach Art einer umlaufenden Dichtlippe. Am Ende 2B ist das Abdichtelement 2 wie das bekannte Abdichtelement 2' ausgebildet, weist also den sich verjüngenden Endbereich 28 auf. Dadurch kann bei dem Abdichtelement 2 gemäß FIG 17 sowohl eine Abdichtung mittels des Abdichtbereichs 20 um die Öffnung 18 auf der Außenfläche der Hornhaut 11 im Bereich 12 als auch eine Abdichtung in der Öffnung 18 zu deren Rand durch den Endbereich 28 oder die Außenfläche des Rüsselbereichs 21 erfolgen.

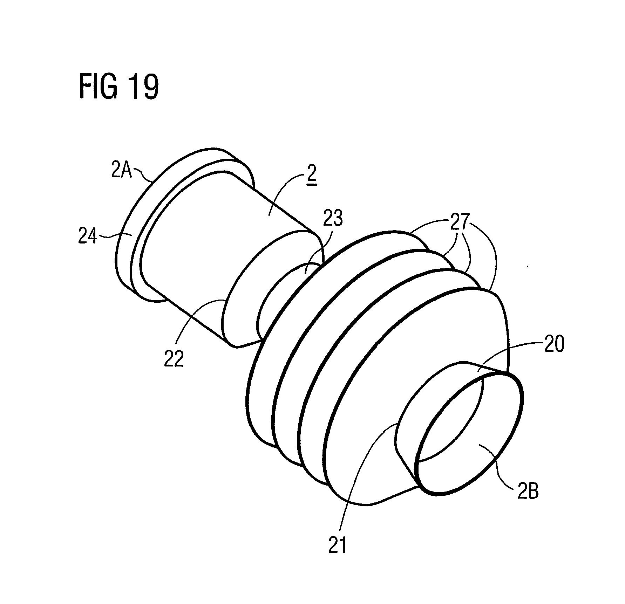

In der weiteren Ausführungsform eines Abdichtelements 2 gemäß FIG 18 und 19 ist ein besonders flexibler Faltenbalg im Bereich des Rüssels 21 vorgesehen. Der Rüssel 21 weist vier umlaufende Falten 27 auf, die unter einem Abstand von a beabstandet sind und deren Flankenbereiche einen Winkel α einschließen. Der Abstand a der Falten 27 kann in einem Bereich zwischen 0,5 mm und 3 mm und 5 mm liegen, insbesondere um 2,5 mm. Der Öffnungswinkel α kann zwischen 20° und 60°, insbesondere bei 30° liegen. Die Wanddicke des Rüssels 21 zumindest im Bereich der Falten 27 kann zwischen 0,1 mm und 0,5 mm, insbesondere bei etwa 0,2 mm gewählt werden. Der Rüssel 21 läuft zum freien Ende 2B hin in einem Abdichtbereich 20 aus, der sich trichterförmig oder konisch nach außen oder zum Ende 2B hin aufweitet unter einem Öffnungswinkel β, der typischerweise zwischen 20° und 60° liegt, beispielsweise zwischen 30° und 40°. Die Wandstärke des Rüssels 21 im Abdichtbereich 20 ist vorzugsweise kleiner als im Bereich der Falten 27 und kann beispielsweise in einem Bereich zwischen 0,05 mm und 0,3 mm, insbesondere bei etwa 0,10 bis 0,15 mm liegen. Dadurch ist der Abdichtbereich 20 noch besonders flexibel und kann sich sehr weich an die Oberfläche der Hornhaut 12 und/oder Sclera 11 anlegen. Ferner ist in FIG 18 noch der Öffnungswinkel γ des konisch zulaufenden Übergangsbereichs 23 eingezeichnet, der im dargestellten Ausführungsbeispiel 120° beträgt, jedoch auch davon abweichen kann.

Zur Beendigung der Kataraktoperation wird nach vollständiger Entfernung der natürlichen Augenlinse und Reinigung des Kapselsackes nun eine künstliche Augenlinse in den Kapselsack eingesetzt und anschließend werden die Wunden verschlossen.

### Bezugszeichenliste

- 2, 2': Abdichtelement
- 2A, 2B: Ende
- 3: Operationsinstrument
- 3A, 3B: Ende
- 5: Handstück

- 10: Auge
- 11: Sclera
- 12: Hornhaut
- 13: Pupille
- 14: Iris
- 15: Augenlinse
- 16: Glaskörper
- 17: vordere Augenkammer
- 18: Öffnung
- 19: Lederhaut
- 20, 20': Abdichtbereich
- 21, 21': Rüssel
- 22, 22': Aufnahmebereich
- 23, 23': Übergangsbereich
- 24, 24': Anschlussflansch
- 25, 25': Durchgang
- 27: Falte
- 28, 28': Abdichtbereich
- 29, 29': Gewinde
- 30: Anschlussbereich
- 50: Irrigationsanschluss
- 51: Anschluss
- α, β, γ: Winkel

## Patentansprüche

1. Vorrichtung zum Abdichten einer Öffnung (18) in einem menschlichen oder tierischen Auge (10) umfassend
wenigstens ein Abdichtelement (2)
a) mit einem von einer Wandung umschlossenen Durchgang (25, 26),
b) und mit wenigstens einem Abdichtbereich (20), der auf einer Außenfläche des die Öffnung umgebenden Augengewebes mit einer die Öffnung vollständig umschließenden Abdichtfläche aufsetzbar oder aufgesetzt ist,
c) wobei durch den Durchgang im Abdichtelement und durch die Öffnung ein Operationsinstrument (3) in den Augeninnenraum (15, 17) durchführbar oder durchgeführt ist, **dadurch gekennzeichnet, dass**
d) der Durchgang im Abdichtelement einen ersten Durchgangsbereich (26) und einen sich an den ersten Durchgangsbereich anschließenden zweiten Durchgangsbereich (25) aufweist, wobei der erste Durchgangsbereich wenigstens teilweise einen Aufnahmeraum zum Aufnehmen eines Handstücks (5) für das Operationsinstrument bildet und das Operationsinstrument durch den zweiten Durchgangsbereich und den Abdichtbereich hindurch geführt oder führbar ist und
e) wobei der erste Durchgangsbereich (26) zumindest überwiegend einen größeren Innenquerschnitt aufweist als der zweite Durchgangsbereich (25).

2. Vorrichtung nach Anspruch 1, bei der die Öffnung operativ erzeugt ist und/oder in der Außenhaut des Auges, insbesondere in der Hornhaut (12) und/oder Sclera (11) und/oder Lederhaut (19), erzeugt ist.

3. Vorrichtung nach einem oder mehreren der vorhergehenden Ansprüche, bei der das Abdichtelement wenigstens im Abdichtbereich elastisch verformbar ist.

4. Vorrichtung nach einem oder mehreren der vorhergehenden Ansprüche, bei der die Abdichtfläche des Abdichtbereichs im unverformten Zustand im Wesentlichen ringförmig ausgebildet ist.

5. Vorrichtung nach einem oder mehreren der vorhergehenden Ansprüche, bei der die Abdichtfläche des Abdichtbereichs im unverformten Zustand im Wesentlichen eben ausgebildet ist.

6. Vorrichtung nach einem oder mehreren der Ansprüche 1 bis 4, bei der die Abdichtfläche des Abdichtbereichs im unverformten Zustand im Wesentlichen gekrümmt in Anpassung an die Krümmung des Augengewebes, insbesondere der Hornhaut, Sclera und/oder Lederhaut, ausgebildet ist.

7. Vorrichtung nach einem oder mehreren der vorhergehenden Ansprüche, bei der der Abdichtbereich sich zur Abdichtfläche hin aufweitet

8. Vorrichtung nach Anspruch 7, bei der der Abdichtbereich sich zur Abdichtfläche hin konkav und/oder trichter- oder trompetenförmig aufweitet.

9. Vorrichtung nach Anspruch 7, bei der der Abdichtbereich sich zur Abdichtfläche hin konvex und/oder glocken- oder kuppelförmig aufweitet.

10. Vorrichtung nach einem oder mehreren der vorhergehenden Ansprüche, bei der eine Abmessung der Abdichtfläche in Richtung zur Öffnung hin und/oder in radialer Richtung und/oder die Dicke der Wandung im Abdichtbereich in einem Bereich zwischen 0,1 mm und 7 mm, insbesondere zwischen 0,2 mm und 1,5 mm, gewählt ist.

11. Vorrichtung nach einem oder mehreren der vorhergehenden Ansprüche, bei der ein maximaler Durchmesser der von der Abdichtfläche umschlossenen Fläche des Augengewebes in einem Bereich zwischen 3 mm und 10 mm gewählt ist.

12. Vorrichtung nach einem oder mehreren der vorhergehenden Ansprüche, bei der ein maximaler Durchmesser der Öffnung im Augengewebe in einem Bereich zwischen etwa 1,5 mm und etwa 6 mm liegt.

13. Vorrichtung nach einem oder mehreren der vorhergehenden Ansprüche, bei der die auf dem Augengewebe aufgesetzte Abdichtfläche des Abdichtbereichs des Abdichtelements von der Öffnung beabstandet ist, insbesondere um einen Minimalabstand aus einem Bereich von 0,1 mm bis 2 mm und/oder bei der der minimale Durchmesser der von der Abdichtfläche umschlossenen Fläche des Augengewebes größer ist als der maximale Durchmesser der Öffnung, insbesondere um einen Betrag aus einem Bereich von 0,1 mm bis 2 mm.

14. Vorrichtung nach einem der vorhergehenden Ansprüche, bei der die Wandung des ersten Durchgangsbereichs zumindest überwiegend dicker ist als die Wandung des zweiten Durchgangsbereichs.

15. Vorrichtung nach einem der vorhergehenden Ansprüche, bei der das Abdichtelement einen Rüsselbereich aufweist, in dem der zweite Durchgangs bereich verläuft.

16. Vorrichtung nach einem der vorhergehenden Ansprüche, bei der die Wandung des zweiten Durchgangsbereichs wenigstens eine umlaufende Falte aufweist oder wie ein Faltenbalg ausgebildet ist.

17. Vorrichtung nach einem oder mehreren der vorhergehenden Ansprüche, bei der der Durchgang oder zweite Durchgangsbereich in dem Abdichtbereich nach außen mündet.

18. Vorrichtung nach einem oder mehreren der vorhergehenden Ansprüche, bei der der Durchgang oder zweite Durchgangsbereich in einem Mündungsbereich nach außen mündet und der Abdichtbereich an der Wandung des Durchgangs ausgebildet ist, insbesondere als umlaufende Dichtlippe.

19. Vorrichtung nach Anspruch 18, bei der im Mündungsbereich des Durchgangs ein zweiter Abdichtbereich vorgesehen ist, der mit einer zweiten Abdichtfläche gegen das Augengewebe an der inneren Randfläche der Öffnung abdichtet oder anliegt.

20. Vorrichtung nach einem oder mehreren der vorhergehenden Ansprüche, bei der das Abdichtelement einstückig ausgebildet ist, insbesondere als Formkörper aus einem Material.

21. Vorrichtung nach einem oder mehreren der vorhergehenden Ansprüche, bei der das Abdichtelement wenigstens teilweise elastisch oder aus einem elastischen Material ausgebildet ist, insbesondere aus einem oder auf Basis eines Naturkautschuk oder Synthesekautschuk oder Elastomer, vorzugsweise Siloxankautschuk.

22. Vorrichtung nach einem oder mehreren der vorhergehenden Ansprüche, bei der der Abdichtbereich zumindest im Bereich der Abdichtfläche eingefärbt oder optisch wenig oder nicht transparent ausgebildet ist.

23. Vorrichtung nach einem oder mehreren der vorhergehenden Ansprüche mit Irrigationsmitteln zum Durchleiten von Irrigationsflüssigkeit durch den Durchgang des Abdichtelements, insbesondere den Zwischenraum zwischen Operationsinstrument und Abdichtelement, und durch die Öffnung in den Augeninnenraum.

24. Vorrichtung nach Anspruch 23, bei der die Irrigationsmittel in einem Handstück integriert sind, an das das Operationsinstrument anschließbar oder angeschlossen ist.

25. Vorrichtung nach einem oder mehreren der vorhergehenden Ansprüche, bei der das Operationsinstrument langgestreckt, insbesondere nadel- oder kanülenförmig, ausgebildet ist und/oder einen Außendurchmesser von maximal 3 mm aufweist.

26. Vorrichtung nach einem oder mehreren der vorhergehenden Ansprüche, bei der das Operationsinstrument ein Ultraschall-Operationsinstrument ist.

27. Vorrichtung nach einem der Ansprüche 1 bis 27, bei der das Operationsinstrument ein photolysisches Operationsinstrument ist.

28. Vorrichtung nach einem der vorhergehenden Ansprüche, bei der das Operationsinstrument ein Operationsinstrument zur Phakolyse, insbesondere Phakoemulsifikation, ist.

29. Vorrichtung nach einem der Ansprüche 1 bis 25, bei der das Operationsinstrument ein Schneid- oder Skalpell-Operationsinstrument ist.

30. Vorrichtung nach Anspruch 29, bei der das Operationsinstrument ein Operationsinstrument zur Vitrektomie ist.

31. Vorrichtung nach einem der Ansprüche 1 bis 25, bei der das Operationsinstrument ein Irrigations- und/oder Manipulationsinstrument ist.

## Claims

1. Device for sealing an opening (18) in a human or animal eye (10) comprising
at least one sealing element (2)
a) having a passageway (25, 26) surrounded by a wall,
b) and at least one sealing region (20) which is or can be placed on an outer surface of the eye tissue surrounding the opening with a sealing surface entirely surrounding the opening,
c) wherein a surgical instrument (3) is or can be introduced through the passageway in the sealing element and through the opening into the eye interior (15, 17), **characterised in that**
d) the passageway in the sealing element comprises a first passageway region (26) and a second passageway region (25) adjoining the first passageway region, wherein the first passageway region at least partially forms a receptacle chamber for receiving a handle (5) for the surgical instrument and the surgical instrument is or can be guided through the second passageway region and the sealing region and
e) wherein the first passageway region (26) has at least predominantly a larger internal cross-section than the second passageway region (25).

2. Device according to claim 1, wherein the opening is surgically created and/or is created in the integument of the eye, particularly in the cornea (12) and/or the sclera (11) and/or the Tunica fibrosa (19).

3. Device according to one or more of the preceding claims wherein
the sealing element is elastically deformable at least in the sealing region.

4. Device according to one or more of the preceding claims wherein the sealing surface of the sealing region is configured essentially annular in the unformed condition.

5. Device according to one or more of the preceding claims, wherein the sealing surface of the sealing region is configured planar in the unformed condition.

6. Device according to one or more of the claims 1 to 4, wherein the sealing surface of the sealing region is formed curved in the unformed condition to adapt to the curvature of the eye tissue, particularly the cornea, sclera and/or Tunica fibrosa.

7. Device according to one or more of the preceding claims, wherein the sealing region broadens toward the sealing surface.

8. Device according to claim 7, wherein the sealing region broadens concavely and/or in a funnel-shaped or trumpet-shaped manner toward the sealing surface.

9. Device according to claim 7, wherein the sealing region broadens convexly and/or in a bell-shaped or bowl-shaped manner toward the sealing surface.

10. Device according to one or more of the preceding claims, wherein a dimension of the sealing surface in the direction toward the opening and/or in the radial direction and/or the thickness of the wall in the sealing region is selected to lie in a range between 0.1 mm and 7 mm, particularly between 0.2 mm and 1.5 mm.

11. Device according to one or more of the preceding claims, wherein a maximum diameter of the area of the eye tissue enclosed by the sealing surface is selected to lie in a range between 3 mm and 10 mm.

12. Device according to one or more of the preceding claims, wherein a maximum diameter of the opening in the eye tissue lies in a range between approximately 1.5 mm and approximately 6 mm.

13. Device according to one or more of the preceding claims, wherein the sealing surface of the sealing region of the sealing element placed on the eye tissue is spaced apart from the opening, particularly by a minimum distance in a range between 0.1 mm and 2 mm and/or wherein the minimum diameter of the area of the eye tissue enclosed by the sealing surface is greater than the maximum diameter of the opening, particularly by an amount in the range between 0.1 mm and 2 mm.

14. Device according to one of the preceding claims, wherein the wall of the first passageway region is at least predominantly thicker than the wall of the second passageway region.

15. Device according to one of the preceding claims, wherein the sealing element has a proboscis region within which the second passageway region extends.

16. Device according to one of the preceding claims, wherein the wall of the second passageway region has at least one peripheral fold or is configured like a bellows.

17. Device according to one or more of the preceding claims, wherein the passageway or the second passageway region opens outwardly in the sealing region.

18. Device according to one or more of the preceding claims, wherein the passageway or the second passageway region opens outwardly in an aperture region and the sealing region is configured on the wall of the passageway, particularly as a peripheral sealing lip.

19. Device according to claim 18, wherein, provided in the aperture region of the passageway, is a second sealing region which seals or lies with a second sealing surface against the eye tissue on the inner peripheral surface of the opening.

20. Device according to one or more of the preceding claims, wherein the sealing element is configured in one piece, particularly as a moulded body made from one material.

21. Device according to one or more of the preceding claims, wherein the sealing element is at least partially elastic or is made from an elastic material, particularly from a natural rubber or a material based on natural rubber or synthetic rubber or an elastomer, preferably siloxane rubber.

22. Device according to one or more of the preceding claims, wherein the sealing region is coloured or configured optically slightly transparent or not transparent, at least in the region of the sealing surface.

23. Device according to one or more of the preceding claims, comprising irrigation means for conducting irrigation fluid through the passageway of the sealing element, particularly through the intermediate space between the surgical instrument and the sealing element, and through the opening into the eye interior.

24. Device according to claim 23, wherein the irrigation means is integrated into a handle to which the surgical instrument can be or is connected.

25. Device according to one or more of the preceding claims, wherein the surgical instrument is configured elongate, particularly needle or cannula-shaped and/or has an outer diameter of not more than 3 mm.

26. Device according to one or more of the preceding claims, wherein the surgical instrument is an ultrasonic surgical instrument.

27. Device according to one of the claims 1 to 27, wherein the surgical instrument is a photolytic surgical instrument.

28. Device according to one of the preceding claims, wherein the surgical instrument is a surgical instrument for phacolysis, particularly phacoemulsification.

29. Device according to one of the claims 1 to 25, wherein the surgical instrument is a cutting or scalpel surgical instrument.

30. Device according to claim 29, wherein the surgical instrument is a surgical instrument for vitrectomy.

31. Device according to one of the claims 1 to 25, wherein the surgical instrument is an irrigation and/or manipulation instrument.

## Revendications

1. Dispositif pour étanchéifier une ouverture (18) dans un oeil (10)
d'un homme ou animal, comprenant
au moins un élément d'étanchéification (2)
a) avec un passage (25, 26) entouré par une paroi,
b) et avec au moins une zone d'étanchéification (20) qui peut ou est placée sur une face extérieure du tissu oculaire entourant l'ouverture avec une face d'étanchéification entourant
complètement l'ouverture,
c) où à travers le passage dans l'élément d'étanchéification et à travers l'ouverture, un instrument d'opération (3) peut ou est inséré dans l'espace intérieur de l'oeil (15, 17), **caractérisé en ce que**
d) le passage dans l'élément d'étanchéification présente une première zone de passage (26) et une deuxième zone de passage (25) faisant suite à la première zone de passage, où la première zone de passage forme au moins partiellement un logement pour la réception d'une pièce à main (5) pour l'instrument d'opération, et l'instrument d'opération peut être ou est guidé à travers la deuxième zone de passage et la zone d'étanchéification et
e) où la première zone de passage (26) présente au moins majoritairement une plus grande section transversale intérieure que la deuxième zone de passage (25).

2. Dispositif selon la revendication 1, dans lequel l'ouverture est réalisée par opération et/ou est réalisée dans la peau extérieure de l'oeil, en particulier dans la cornée (12) et/ou scléra (11) et/ou l'enveloppe dure (19).

3. Dispositif selon une ou plusieurs des revendications précédentes,
dans lequel l'élément d'étanchéification est déformable
élastiquement au moins dans la zone d'étanchéification.

4. Dispositif selon l'une ou plusieurs des revendications précédentes, dans lequel la face d'étanchéification de la zone d'étanchéification, à l'état non déformé, est sensiblement annulaire.

5. Dispositif selon une ou plusieurs des revendications précédentes, dans lequel la face d'étanchéification de la zone d'étanchéification, à l'état non déformé, est sensiblement plane.

6. Dispositif selon une ou plusieurs des revendications 1 à 4, dans lequel la face d'étanchéification de la zone d'étanchéification, à l'état non déformé, est sensiblement courbée, en adaptation à la courbure du tissu oculaire, en particulier de la cornée, scléra et/ou enveloppe dure.

7. Dispositif selon l'une ou plusieurs des revendications précédentes, dans lequel la zone d'étanchéification s'élargit vers la face d'étanchéification.

8. Dispositif selon la revendication 7, dans lequel la zone d'étanchéification s'élargit vers la face d'étanchéification d'une manière concave et/ou en forme d'entonnoir ou de trompette.

9. Dispositif selon la revendication 7, dans lequel la zone d'étanchéification s'élargit vers la face d'étanchéification d'une manière concave et/ou en forme de cloche ou de coupole.

10. Dispositif selon une ou plusieurs des revendications précédentes, dans lequel une dimension de la face d'étanchéification, en direction de l'ouverture et/ou dans la direction radiale et/ou l'épaisseur de la paroi dans la zone d'étanchéification est sélectionnée dans une plage entre 0,1 mm et 7 mm, en particulier entre 0,2 mm et 1,5 mm.

11. Dispositif selon une ou plusieurs des revendications précédentes, dans lequel un diamètre maximal de la face entourée par la face d'étanchéification du tissu oculaire est sélectionné dans une plage entre 3 mm et 10 mm.

12. Dispositif selon une ou plusieurs des revendications précédentes, dans lequel un diamètre maximal de l'ouverture dans le tissu oculaire se situe dans une plage entre environ 1,5 mm et environ 6 mm.

13. Dispositif selon une ou plusieurs des revendications précédentes, dans lequel la face d'étanchéification appliquée au tissu oculaire de la zone d'étanchéification de l'élément d'étanchéification est espacée de l'ouverture, en particulier d'un écart minimal d'une plage de 0,1 mm à 2 mm et/ou dans lequel le diamètre minimal de la face entourée par la face d'étanchéification du tissu oculaire est plus grand que le diamètre maximal de l'ouverture, en particulier d'une valeur d'une plage de 0,1 mm à 2 mm.

14. Dispositif selon l'une des revendications précédentes, dans lequel la paroi de la première zone de passage est au moins majoritairement plus épaisse que la paroi de la deuxième zone de passage.

15. Dispositif selon l'une des revendications précédentes, dans lequel l'élément d'étanchéification présente une zone de museau dans laquelle s'étend la deuxième zone de passage.

16. Dispositif selon l'une des revendications précédentes, dans lequel la paroi de la deuxième zone de passage présente au moins un pli s'étendant tout autour ou est réalisé comme un soufflet.

17. Dispositif selon une ou plusieurs des revendications précédentes, dans lequel le passage ou la deuxième zone de passage dans la zone d'étanchéification débouche vers l'extérieur.

18. Dispositif selon une ou plusieurs des revendications précédentes, dans lequel le passage ou la deuxième zone de passage dans une zone d'embouchure débouche vers l'extérieur, et la zone d'étanchéification est réalisée à la paroi du passage, en particulier comme lèvre d'étanchéité s'étendant tout autour.

19. Dispositif selon la revendication 18, dans lequel est prévue dans la zone d'embouchure du passage une deuxième zone d'étanchéification qui rend étanche ou s'applique avec une deuxième face d'étanchéification au tissu oculaire à la face de bord intérieure de l'ouverture.

20. Dispositif selon une ou plusieurs des revendications précédentes, dans lequel l'élément d'étanchéification est réalisé en une pièce, en particulier en tant que corps moulé en un matériau.

21. Dispositif selon une ou plusieurs des revendications précédentes, dans lequel l'élément d'étanchéification est réalisé au moins partiellement d'une manière élastique ou en un matériau élastique, en particulier en un caoutchouc naturel ou caoutchouc synthétique ou élastomère ou à base de ceux-ci, de préférence en caoutchouc siloxane.

22. Dispositif selon une ou plusieurs des revendications précédentes, dans lequel la zone d'étanchéification, au moins dans la zone de la face d'étanchéification, est colorée ou est réalisée optiquement peu ou non transparente.

23. Dispositif selon une ou plusieurs des revendications précédentes avec des moyens d'irrigation pour faire passer un liquide d'irrigation à travers le passage de l'élément d'étanchéification, en particulier l'espace intermédiaire entre l'instrument d'opération et l'élément d'étanchéification, et à travers l'ouverture dans l'espace intérieur de l'oeil.

24. Dispositif selon la revendication 23, dans lequel les moyens d'irrigation sont intégrés dans une pièce à main à laquelle est ou peu être connecté l'instrument d'opération.

25. Dispositif selon une ou plusieurs des revendications précédentes, dans lequel l'instrument d'opération est réalisé d'une manière allongée, en particulier en forme d'aiguille ou de canule et/ou présente un diamètre extérieur de 3 mm au maximum.

26. Dispositif selon une ou plusieurs des revendications précédentes, dans lequel l'instrument d'opération est un instrument d'opération à ultrasons.

27. Dispositif selon l'une des revendications 1 à 27, dans lequel l'instrument d'opération est un instrument d'opération photolytique.

28. Dispositif selon l'une des revendications précédentes, dans lequel l'instrument d'opération est un instrument d'opération pour la phakolyse, en particulier une phako-émulsification.

29. Dispositif selon l'une des revendications 1 à 25, dans lequel l'instrument d'opération est un instrument de coupe ou un scalpel.

30. Dispositif selon la revendication 29, dans lequel l'instrument d'opération est un instrument d'opération pour la vitrectomie.

31. Dispositif selon l'une des revendications 1 à 25, dans lequel l'instrument d'opération est un instrument d'irrigation et/ou de manipulation.
